# EUROPÄISCHE PATENTSCHRIFT

(11) **EP 2 609 173 B1**
(45) Veröffentlichungstag und Bekanntmachung des Hinweises auf die Patenterteilung: **28.02.2018**
(21) Anmeldenummer: 11748333.9
(22) Anmeldetag: 05.08.2011
(51) Int. Cl.: C09K 21/14, C08G 79/02, C08K 13/02

(54) **FLAMMSCHUTZMITTELZUSAMMENSETZUNGEN ENTHALTEND TRIAZIN-INTERKALIERTE METALL-PHOSPHATE**
FLAME PROTECTION AGENT COMPOSITIONS CONTAINING TRIAZINE INTERCALATED METAL PHOSPHATES
COMPOSITIONS IGNIFUGES CONTENANT UN PHOSPHATE MÉTALLIQUE À TRIAZINE INTERCALÉE

(30) Priorität: 23.08.2010 DE 102010035103
(43) Veröffentlichungstag der Anmeldung: 03.07.2013
(60) Teilanmeldung: 17202455.6
(73) Patentinhaber: J.M. Huber Corporation, Atlanta, GA 30339 (US)
(72) Erfinder: KÖSTLER, Hans-Günter, 64646 Heppenheim (DE); DAVE, Trupti, 64673 Zwingenberg (DE); WEHNER, Wolfgang, 64673 Zwingenberg (DE)
(74) Vertreter: Murgitroyd & Company
(86) Internationale Anmeldenummer: PCT/EP2011/063567
(87) Internationale Veröffentlichungsnummer: WO 2012/025362

(56) Entgegenhaltungen:
- WO-A2-02/48248
- DE-A1-102007 036 465
- HIROKAZU NAKAYAMA: "INTERCALATION OF ORGANIC MOLELECULES INTO LAYERED PHOSPHATES", PHOSPHORUS RESEARCH BULLETIN, Bd. 23, 1. Januar 2009 (2009-01-01), Seiten 1-9, XP55017083, ISSN: 0918-4783, DOI: 10.3363/prb.23.1
- JENNY ALONGI ET AL: "Flame retardancy properties of [alpha]-zirconium phosphate based composites", POLYMER DEGRADATION AND STABILITY, Bd. 95, Nr. 9, 27. April 2010 (2010-04-27) , Seiten 1928-1933, XP55017029, ISSN: 0141-3910, DOI: 10.1016/j.polymdegradstab.2010.04.007
- "Melamine derivatives", SpecialChem S.A. , 28. Juni 2009 (2009-06-28), XP002667710, Gefunden im Internet: URL:http://web.archive.org/web/20090628141 703/http://www.specialchem4polymers.com/tc /Melamine-Flame-Retardants/index.aspx?id=4 004 [gefunden am 2012-01-20]

## Beschreibung

Die vorliegende Erfindung betrifft Flammschutzmittelzusammensetzungen enthaltend Triazin-interkalierte Metall-Phosphate mit offenen Gerüststrukturen (Open Framework), deren Verwendung, solche Metallphosphate sowie deren Herstellung.

Es ist bekannt, organophile Schichtsilikate, welche beispielsweise mittels Ionenaustausch hergestellt wurden, als Füllmaterialien für thermoplastische Werkstoffe sowie für Duroplaste zu verwenden, wobei Nanocomposite erhalten werden. Bei der Verwendung von geeigneten organophilen Schichtsilikaten als Füllmaterialien werden die physikalischen und mechanischen Eigenschaften der derart hergestellten Formteile erheblich verbessert. Von besonderem Interesse ist die Erhöhung der Steifigkeit bei mindestens gleichbleibender Zähigkeit. Besonders gute Eigenschaften zeigen Nanocomposite, welche das Schichtsilikat in exfoliierter Form enthalten. Diese Nanocomposite werden bevorzugt als Flammschutzmittel bzw. als Synergisten eingesetzt.

Aus WO-A 00/44669 sind organophile Schichtsilikate bekannt, die durch Behandlung eines natürlichen oder synthetischen Schichtsilikats oder eines Gemisches solcher Silikate, mit einem Salz einer - gegebenenfalls quaternären - cyclischen Melaminverbindung oder einem Gemisch solcher Salze, hergestellt werden.

Ähnliche Überlegungen sollten auch für organophile Metall-Phosphate mit offenen Gerüststrukturen gelten (s. hierzu Def. "A Review of Open Framework Structures", Annu. Rev. Mater. Sci. 1996, 26, 135-151), insbesondere für jene, die mit Melamin interkaliert sind (Interkalat auch Einlagerungsverbindung genannt, s. hierzu Def. in RÖMPP, Chemielexikon, 9.Aufl., 1995 ,G. Thieme, Bd. 3, S2005).

In der Literatur sind diverse Melamin-Phosphate beschrieben, die keine offenen Gerüststrukturen aufweisen. So Melamin-ortho-Phosphat in Magn. Reson. Chem. 2007, 45, S231-S246., Bis-Melamin-di(pyro)-Phosphat in J. Phys. Chem. B 2004, 108, 15069-15076 und Melamin-poly-Phosphat in J. Phys. Chem. B 2005, 109, 13529-13537. Deren Verwendung als Flammschutzmittel ist dort in der zitierten Sekundärliteratur aufgeführt.

Gewisse Melamin-Metall-Phosphate sind beschrieben in WO-A 2009/015772. Diese Verbindungen besitzen aber - wie an der Aluminiumverbindung gezeigt - nur eine begrenzte (Thermo)Eigenstabilität, die für eine Einarbeitung in Polyamiden nicht ausreichend ist (siehe Beispiele 7 und 8).

Melamin-interkalierte Zirkonium-(Schicht)-Phosphate sind bekannt aus Solid State Sciences 2009, 11, 1007-1015. Die Verwendung als Kunststoffadditive, insbesondere als Flammschutzmittel ist hierin aber nicht beschrieben. Andere Melamin-interkalierte (Metall)Schichtphosphate sind in der Literatur nicht belegt.

Eine Interkalation von α, ω-Alkandiaminen in Aluminium-(Schicht) Triphosphat ist publiziert in J. Inclusion Phenomena andMacrocyclic Chem. 1999, 34 401-412.

Die Schichtstruktur von Aluminiumtriphosphat wird in Chem. Commun. 2006, 747-749 belegt. Eine offene Netzstruktur ist für Ethylendiamin-Zinkphosphat-Addukte aus Zeolites and Related Microporous Materials 1994, 2229-2236 bekannt.

Ethylendiamin-bis-zinkphosphat ist in US 5994435 und US6207735 als Flammschutzmittel beansprucht. In JP 8269230 sind Amin-Zinkphosphat beschrieben, die auch die Anionen HPO₄, H₂PO₄, Zn₂(HPO₄)₃ und Zn₄[(PO₄)₂ (HPO₄)₂] umfassen. Die Anmeldungen JP9040686, JP10259275, JP11152373, JP11199708, JP11246754, JP11269187, JP11293155, JP2000063562, JP2000063563, JP2000154283, JP2000154287, JP2000154324 und JP2001031408 beschreiben Verfahren zur Herstellung spezieller Anwendungsformen sowie Kombinationen von Ethylendiamin-Zinkphosphat. Die Verfahren sind jedoch unwirtschaftlich, da sie entweder mit H₃PO₄-Überschuss arbeiten oder von Zn(en)₃-Komplexen ausgehen. JP9169784 und JP2001011462 publizieren Diethylentriamin- bzw. Piperazin-Zinkphosphat-Komplexe als Flammschutzmittel.

Anorganische Phosphate mit offenen Gerüststrukturen sind in einem Aufsatz in Angew. Chem. 1999, 111, 3466-3492 beschrieben.

Nachteilig an den genannten Verbindungen aus dem Stand der Technik sind die begrenzte (Thermo)-Eigenstabilität und die nach Einarbeitung ins Polymersubstrat resultierenden ungünstigen mechanischen Eigenschaften.

Die Aufgabe besteht darin, Flammschutzmittelzusammensetzungen bereitzustellen, die ein hohes Maß an (Thermo)-Eigenstabilität aufweisen und dem Polymeren nach Einarbeitung hervorragende mechanische Eigenschaften verleihen.

Die Aufgabe wurde unter anderem gelöst durch die Bereitstellung von Flammschutzmittelzusammensetzungen enthaltend
(a) mindestens ein Triazin-interkaliertes Metall-Phosphat mit mindestens einer Monomereinheit der folgenden allgemeinen Formel (I)

   (A-H)ₐ⁽⁺⁾[M_{b}^{m+}(H₂PO₄)ₓ₁⁽⁻⁾(HPO₄)ₓ₂²⁽⁻⁾(PO₄)ₓ₃³⁽⁻⁾(PO₃)_{y}⁽⁻⁾]^{(a-)}*pH₂O (I)

   wobei
   (A - H)⁽⁺⁾ ein Triazin-Derivat der Formeln (II-1), (II-2) oder (II-3) 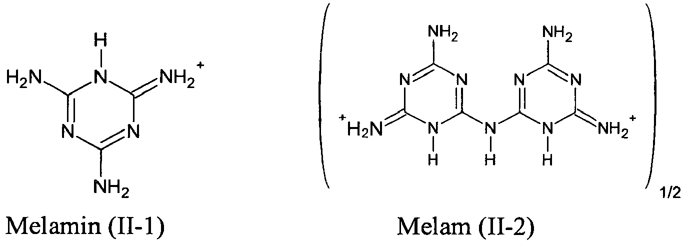 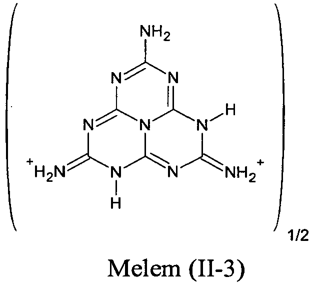 ist;
   jedes **M** = Al ist;
   **a** ist 2,
   **b** ist 1,
   **m** = 3,
      und
   **x₁** = 0 oder 1; **x₂** = 0 oder 2; **x₃** = 1 oder 0; **y** = 2 oder 0 und p = 0 bis 5,
   wobei: a + mb = x₁ + 2x₂ + 3x₃ + y
      und
(b) mindestens eine weitere von (a) verschiedene Flammschutzmittelkomponente.

In einer bevorzugten Ausführungsform der Erfindung weisen die Flammschutzmittelzusammensetzungen enthaltend die Triazin-interkalierten Metall-Phosphate (a) der Formel (I) offene Gerüststrukturen auf. Die Triazinderivate sind ebenso wie Melon als chemische Vorstufen für Kohlenstoff-Nitrid (C₃N₄)ₓ bekannt.

Triazin-interkalierte Metall-Phosphate, insbesondere mit offenen Gerüststrukturen, welche vorzugsweise durch direkte Umsetzung von (wässrigen) aciden Metall-Phosphaten mit Melamin und nachfolgender Temperung aus den entsprechenden (Precursor) Vorläuferstufen hergestellt werden, zeigen eine hohe Thermostabilität bei der Verarbeitung kombiniert mit ausgezeichneter Dispergierwirkung und Grenzflächenadhäsion. Diese Systeme zeichnen sich durch überraschend gute Schichtseparation aus, verbunden mit ausgezeichneter Adhäsion zu einer Vielzahl von Polymeren und Füllstoffen. Weiterhin überraschend ist, dass die erfindungsgemäßen Triazin-interkalierten Metall-Phosphate mit offenen Gerüststrukturen nicht nur hervorragende Füllstoffe zur Verbesserung der mechanischen Eigenschaften von Polymeren sind, sondern auch als Flammschutzmittel wirken. Die Triazin-interkalierten (Metall)Phosphate mit offenen Gerüststrukturen können auch aus Ketten(Band)-Phosphaten (Catena-Typ), Blattphosphaten (Leiter- oder Phyllo-Typ - alle mit 1-D-Strukturen), Schichtphosphaten (layered phosphates - mit 2-D-Strukturen) oder 3-D-Phosphaten (Zeolith-Typ) bestehen.

In einer besonders bevorzugten Ausführungsform der vorliegenden Erfindung ist in den Flammschutzmittelzusammensetzungen enthaltend die Komponente (a), (A-H)⁽⁺⁾ = (II-1) und M= Al.

Vorzugsweise ist die Komponente (b) mindestens eine Metallverbindung, die kein Metallphosphat der Komponente (a) ist, oder/und mindestens eine metallfreie Phosphorverbindung.

Diese mindestens eine Metallverbindung (b) ist vorzugsweise ein Metalloxid, ein Metallhydroxid, ein Metallphosphat, ein Metallpyrophosphat, ein Hydrotalcit, ein kationisch oder anionisch modifizierter Organoclay, ein Stannat oder Molybdat-Salz, ein Metallborat oder Metall-Phosphinat der Formel (III): 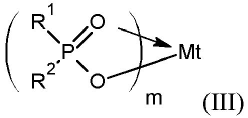 wobei R¹ und R² Wasserstoff oder ein geradkettiger oder ein verzweigter C₁ - C₆Alkylrest oder ein Phenylrest; und Mt = Ca, Mg, Zn oder Al und m = 2 oder 3 bzw. ein Hypophosphitsalz der Formel M^{m+}[H₂PO₂]ₘ^{m-} (M = Al, Ca, Mg und Zn sowie m = 2 und 3) ist.

Unter Organoclays versteht man organophilmodifizierte Tonmineralien (haupsächlich Montmorillonite ) auf Basis von Kationenaustausch wie Triethanol-Talg-Ammonium-Montmorillonit und Triethanol-Talg-Ammonium-Hektorit (Dr. G. Beyer; Konf. Fire Resistance in Plastics 2007*).* Anionic Organoclays bedeuten organophil-modifizierte Hydrotalcite auf Basis von Anionenaustausch mit Alkalirosinaten, ungesättigten und gesättigten Fettsäuresalzen sowie langkettig alkylsubstituierten Sulfonaten und Sulfaten.

Besonders bevorzugt als Metalloxid ist Diantimontrioxid, Diantimontetroxid, Diantimonpentoxid oder Zinkoxid.

Besonders bevorzugt als Metallhydroxid sind Aluminiumhydroxid (ATH) bzw. Gibbsit (Hydrargillit), Aluminiumoxohydroxid (Boehmit) und Magnesiumhydroxid (MDH, Brucit) sowie Hydromagnesit. Neben Gibbsit und Boehmit sind auch die anderen Modifikationen von Aluminiumhydroxiden, nämlich Bayerit, Nordstrandit und Diaspor anzuführen.

Als Metallphosphat sind Metallpyrophosphate bevorzugt. Besonders bevorzugt sind Aluminium- und Zinkpyrophosphat sowie Zink- und Aluminiumtriphosphat ebenso wie Aluminium-metaphosphat und Aluminium-orthophosphat.

Als Hydrotalcit bevorzugt sind Magnesium-Aluminium- und Calcium-Aluminium-Hydroxocarbonat.

Unter den kationisch- oder anionisch-modifizierten Organoclays sind die alkylsulfat- oder fettsäurecarboxylat- modifizierten Hydrotalcite oder langkettig quaternär-ammoniummodifizierten Tonmineralien besonders bevorzugt.

Unter den Stannat- und Molybdatsalzen sind Zinkstannat, Zinkhydroxystannat, Ammoniumheptamolybdat und Ammoniumocatmolybdat besonders bevorzugt. Ebenso zu benennen sind andere Molybdate (auch Polymolybdate) wie Calciumzinkmolybdat, basisches Zinkmolybdat und Calciummolybdat.

Als Borate bevorzugt sind Alkali- und Erdalkaliborate sowie Zinkborat. Weiterhin anzu führen sind Aluminiumborat, Bariumborat, Calciumborat, Magnesiumborat, Manganborat, Melaminborat, Kaliumborat sowie Zinkborphosphat.

Unter den Metallphosphinaten sind bevorzugt Ca-, Mg-, Zn- oder Al-Phosphinate. Besonders bevorzugt sind Ca-, Mg-, Zn- oder Al-Phenyl(benzol)phosphinat und Ca-, Mg-, Zn- oder Al- diethyl(ethan)phosphinat.

Unter den Hypophosphiten sind besonders bevorzugt, das Mg-, Ca-, Zn und Al-Salz.

Eine weitere Bevorzugung der Erfindung betrifft Flammschutzmittelzusammensetzungen enthaltend als Komponente (b) mindestens eine metallfreie Phosphorverbindung.

Diese mindestens eine metallfreie Phosphorverbindung (b) ist roter Phosphor, ein oligomerer Phosphatester, ein oligomerer Phosphonatester, ein zyklischer Phosphonatester, ein Thiopyrophosphorsäureester, Melaminpyrophosphat, Melaminpolyphosphat, Ammoniumpolyphosphat, Melaminium-Phenylphosphonat sowie dessen Halbestersalz (WO2010/063623), Melamin-Benzolphosphinat (WO2010/057851), Hydroxyalkyl-Phosphinoxide (WO2009/034023), Tetrakis-hydroxymethyl-Phosphoniumsalze und Phospholan- bzw. Phosphol-Derivate sowie Bisphosphoramidate mit Piperazin als Brückenglied oder ein Phosphonitester.

Oligomere Phosphatester besitzen Formel (IV) oder Formel (V): 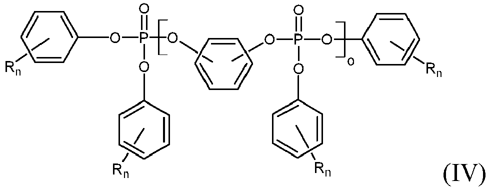 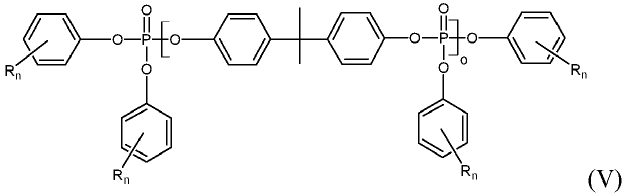 wobei jedes R jeweils unabhängig Wasserstoff, C₁ - C₄ -Alkyl oder Hydroxy, n = 1 bis 3 und o 1 bis 10 ist.

Besonders bevorzugt sind das Oligomer mit Rₙ = H und Resorcin bzw. Hydrochinon als Bestandteil des Brückengliedes sowie Rₙ = H und Bisphenol-A oder Bisphenol-F als Bestandteil des Brückengliedes.

Oligomere Phosphonatester sind vorzugsweise durch Formel (VI) charakterisiert: 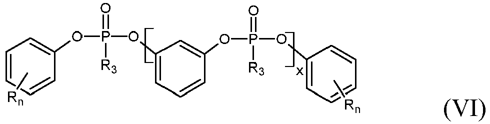 wobei R³ = Methyl oder Phenyl und x 1 bis 20 ist und R, n die oben angegebene Bedeutung besitzen.

Besonders bevorzugt sind das Oligomer mit Rₙ = H und Resorcin bzw. Hydrochinon als Bestandteil des Brückengliedes.

Zyklische Phosphonatester weisen vorzugsweise folgende Formel (VII) auf: 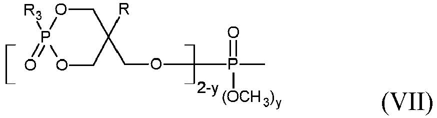 wobei y = 0 oder 2 ist. Besonders bevorzugt ist Bis[5-ethyl-2-methyl-1,3,2-dioxaphosphorinan-5-yl)methyl]methyl phosphonate P,P'-dioxid.

Thiopyrophosphorsäureester sind vorzugsweise durch folgende Formel (VIII) charakterisi ert:

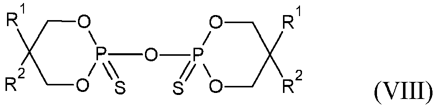

Besonders bevorzugt ist 2,2'-Oxybis[5,5-dimethyl-1,3,2-dioxaphosphorinan]2,2'-disulfid.

Unter den Hydroxyalkyl-Phosphinoxiden sind bevorzugt Isobutyl-bis-hydroxymethyl-Phosphinoxid sowie dessen Kombination mit Epoxyharzen (WO-A 2009/034023).

Unter den Tetrakis-hydroxyalkyl-Phosphoniumsalzen sind die Tetrakis-hydroxymethyl-Phosphoniumsalze besonders bevorzugt.

Unter den Phospholan- bzw. Phosphol-Derivaten sind Dihydrophosphol(oxid)-Derivate und Phospholan (oxid)-derivate sowie deren Salze (EP 089 296 und EP 1024 166) besonders bevorzugt.

Besonders bevorzugt unter den Bisphosphoramidaten sind die Bis-di-ortho-xylylester mit Piperazin als Brückenglied.

Unter den Phosphonitestern sind bevorzugt, Benzol-Phosphinsäurephenylester und dessen PH-fuktionalisierte Derivate und DOPO-Derivate.
Als DOPO-Derivate (9,10-Dihydro-9-oxa-10-Phosphaphenanthren-10-oxid-Derivate oder 6H-Dibenzo(c,e) (1,2)-oxaphosphorin-6-oxid)-Derivate (wobei PH-funktionalisierte Derivate bevorzugt sind) sind folgende Verbindungen strukturell dargestellt (vgl. WO-A 2008/119693):

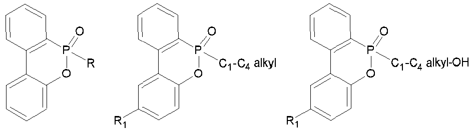

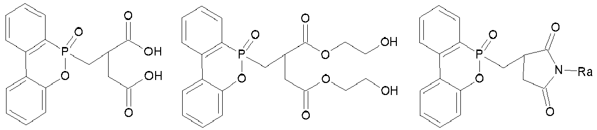

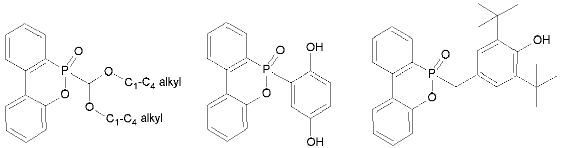

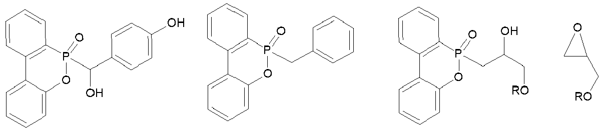

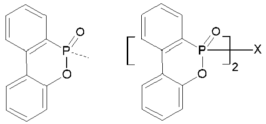

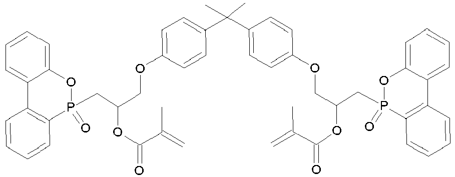

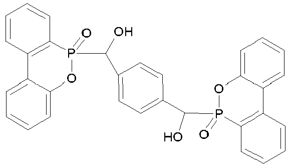

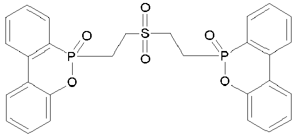

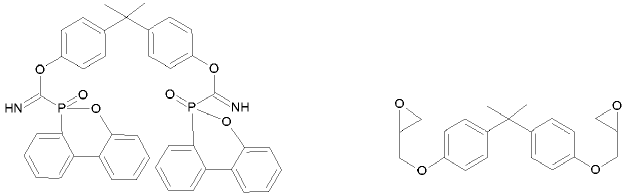

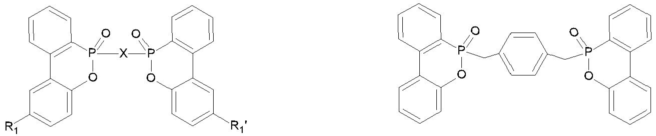

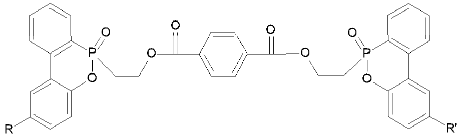

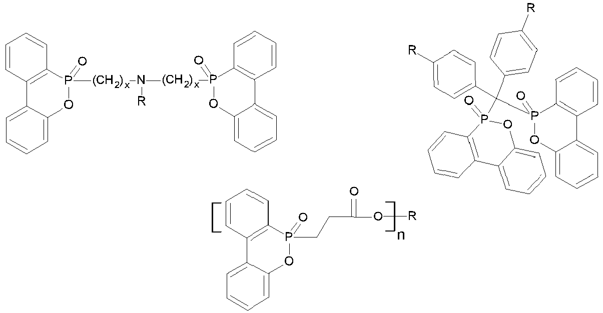

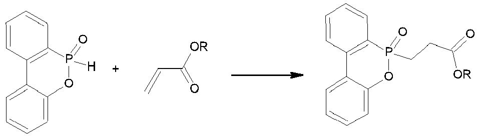

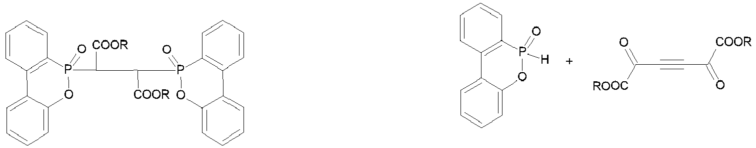

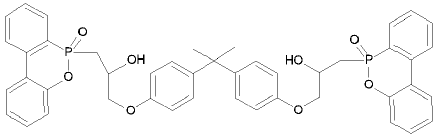

Besonders bevorzugt sind:

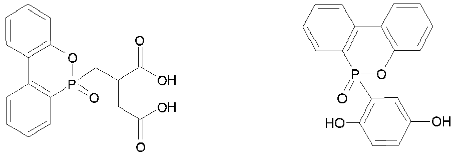

Anstelle von DOPO kann auch Dihydro-oxa-phospha-anthracenoxid(on) treten. Eine Übersicht hierzu ist aus WO-A 2008/119693 zu entnehmen.

Als weitere Additive (Synergisten) sind zu nennen: Polyole, Aminouracile, Trishydroxyethylisocyanurat (THEIC), Melamin(iso)cyanurat, POSS-Verbindungen und Blähgraphit.

Von den Polyolen sind Pentaerythrit, Dipentaerythrit und Tripentaerythrit besonders bevorzugt.

Von den Aminouracilen sind 1-Methyl-6-aminouracil und 1,3-Dimethyl-6-aminouracil besonders bevorzugt.

POSS-Verbindungen (**P**olyhedral **o**ligomeric **S**il**s**esquioxanes) und Derivate werden näher beschrieben in POLYMER, Vol. 46, pp 7855-7866. Bevorzugt sind hierbei POSS-Derivate auf Methylsiloxan-Basis.

Ferner könne auch Tris-hydroxyethyl-isocyanurat-polyterephthalate sowie Triazin-Polymere mit Piperazin- 1,4-diyl-Brückengliedern und Morpholin-1-yl-Endgruppen zugegen sein.

Weiterhin können folgende Zusatzstoffen zugegen sein: Bis-Azinpentaerythritdiphosphat-Salze, Hexa-aryloxy-triphosphazene, Poly-aryloxy-phosphazene und Siloxane (R₂SiO)ᵣ oder (RSiO_{1,5})ᵣ.

Metalloxide wie Titandioxid, Siliziumdioxid; Tonmineralien wie Kaolinit, Muskovit, Pyrophyllit, Bentonit und Talk oder andere Mineralien wie Wollastonit, Quarz, Glimmer, Feldspat.

Ferner können im Polymeren zusätzlich Dolomit, Bentonit, Huntit, oder Kieselsäuren und deren natürliche bzw. synthetische Silikatminerale enthalten sein.

Außerdem können einem Polymer zusätzlich zu dem mindestens einen erfindungsgemäßen Metall-Phosphat Schaumbildner zugesetzt werden. Als Schaumbildner seien genannt: Melamin, Melaminformaldehydharze, Harnstoffderivate wie Harnstoff, Thioharnstoff, Guanamine, Benzoguanamin, Azetoguanamin und Succinylguanamin, Dizyandiamid, Guanidin und Guanidinsulfamat sowie andere Guanidinsalze bzw. Allantoine und Glykolurile.

Darüber hinaus kann ein Polymer, enthaltend das mindestens eine erfindungsgemäße Metall-Phosphat, auch Antidripping-Mittel, insbesondere auf Polytetrafluoroethylen-Basis enthalten. Die Konzentration solcher Antidrippingmittel beträgt 0,01 bis 15 Gew.-% bezogen auf das zu verarbeitende Polymer.

Zusätzlich können Polymeren, enthaltend das mindestens eine erfindungsgemäße Metall-Phosphat, auch weitere Komponenten zugegeben werden, wie z.B. Füllstoffe und Verstärkungsmittel, wie Glasfasern, Glasperlen oder mineralische Zusätze wie Kreide. Als weitere Additive können Antioxidantien, Lichtstabilisatoren, Gleitmittel, Pigmente, Nukleierungsagentien und Antistatika fungieren.

Die vorliegende Erfindung betrifft außerdem die Verwendung der erfindungsgemäßen Triazin-interkalierten Metall-Phosphate mit offenen Gerüststrukturen als Flammschutzmittel in einem Polymer, Papier, Textilien oder Wood Plastic Composites (WPC).

Die erfindungsgemäßen Flammschutzmittel sind bestens geeignet, synthetischen, insbesondere thermoplastischen Polymeren, Flammschutzeigenschaften zu verleihen.

Eine besondere Ausführungsform der Erfindung betrifft die Verwendung des mindestens einen erfindungsgemäßen Metall-Phosphats in einem Polymer als Flammschutzmittel, wobei das Polymer ein Thermoplast der vorzugsweise ausgewählt ist aus der Gruppe bestehend aus Polyamid, Polycarbonat, Polyolefin, Polystyrol, Polyester, Polyvinylchlorid, Polyvinylalkohol, ABS und Polyurethan oder ein Duroplast ist, der vorzugsweise ausgewählt ist aus der Gruppe bestehend aus Epoxidharz (mit Härter), Phenolharz und Melaminharz.

Ist das Polymer, in dem das mindestens eine erfindungsgemäße Metall-Phosphat als Flammschutzmittel eingesetzt wird, ein Thermoplast, so ist Polyamid, Polyurethan, Polystyrol, Polyolefin oder Polyester bevorzugt.

Ist das Polymer, in dem das mindestens eine erfindungsgemäße Metall-Phosphat als Flammschutzmittel eingesetzt wird, ein Duroplast, so ist Epoxidharz bevorzugt.

Es können auch Mischungen von einem oder mehreren Polymeren, insbesondere Thermo- und/oder Duroplasten, in denen das erfindungsgemäße Metall-Phosphat als Flammschutzmittel eingesetzt wird, verwendet werden.

Beispiele für solche Polymere sind:
1) Polymere von Mono- und Diolefinen, z.B. Polypropylen, Polyisobutylen, Polybuten-1, Poly-4-methylpenten-1, Polyvinylcyclohexan, Polyisopren oder Polybutadien und Polymerisate von Cycloolefinen, z.B. von Cyklopenten oder Norbornen und Polyethylen (auch vernetzt), z.B. High Density Polyethylen (HDPE) oder High Molecular Weight (HDPE-HMW), High Density Polyethylen mit Ultra-High Molecular Weight (HDPE-UHMW), Medium Density Polyethylen (MDPE), Low Density Polyethylen (LDPE) und Linear Low Density Polyethylen (LLDPE), (VLDPE) und (ULDPE) sowie Copolymere von Ethylen und Vinylacetat (EVA);
2) Polystyrole, Poly(p-methylstyrol), Poly(a-methylstyrol);
3) Copolymere sowie Propfcopolymere von Polybutadien-Styrol oder Polybutadien und (Meth)Acrylnitril wie z.B. ABS und MBS;
4) Halogenhaltige Polymere z.B. Polychloropren, Polyvinylchlorid (PVC); Polyvinylidenchlorid (PVDC), Copolymere von Vinylchlorid/Vinylidenchlorid, Vinylchlorid/Vinylacetat oder Vinylchlorid/Vinylacetat;
5) Poly(meth)acrylate, Polymethylmethacrylate (PMMA), Polyacrylamid und Polyacrylnitril (PAN);
6) Polymere von ungesättigten Alkoholen und Aminen oder ihren Azylderivaten bzw. Azetalen, wie z.B. Polyvinylalkohol (PVA), Polyvinylacetate, -stearate, -benzoate oder maleate, Polyvinylbutyral, Polyallylphthalate und Polyallylmelamine;
7) Homo- und Copolymere von cyclischen Ethern, wie Polyalkylenglykole, Polyethylenoxide, Polypropylenoxide und deren Copolymere mit Bisglycidylethern;
8) Polyazetale, wie Polyoxymethylene (POM) sowie Polyurethan und Acrylat modifizierte Polyazetale;
9) Polyphenylenoxide und -sulfide und deren Gemische mit Styrolpolymeren oder Polyamiden;
10) Polyamide und Copolyamide hergeleitet von Diaminen und Dicarbonsäuren und/oder von Aminocarbonsäuren oder den entsprechenden Laktamen, wie z.B. Polyamid 4, Polyamid 6, Polyamid 6/6, 6/10, 6/9, 6/12, 12/12, Polyamid 11, Polyamid 12, aromatische Polyamide hergeleitet vom m-Xylylendiamin und Adipinsäure und Copolyamide modifiziert mit EPDM oder ABS. Beispiele von Polyamiden und Copolyamiden sind hergeleitet von ε-Kaprolaktam, Adipinsäure, Sebacinsäure, Dodekansäure, Isophthalsäure, Terephthalsäure, Hexamethylendiamin, Tetramethylendiamin, 2-Methyl-pentamethylendiamin, 2,2,4-Trimethylhexamethylendiamin, 2,4,4-Trimethylhexamethylendiamin, m-Xylylendiamin oder Bis(3-Methyl-4-aminozyklohexyl)methan;
11) Polyharnstoffe, Polyimide, Polyamidimide, Polyetherimide, Polyesterimide, Polyhydantoine und Polybenzimidazole;
12) Polyester hergeleitet von Dicarbonsäuren und Dialkoholen und/oder Hydroxycarbonsäuren oder den entsprechenden Laktonen, wie z.B. Polyethylenterephthalat, Polypropylenterephthalat, Polybutylenterephthalat, Poly-1,4-dimethylzyklohexanterephthalat, Polyalkylennaphthalat (PAN) und Polyhydroxybenzoate, Polymilchsäureester und Polyglykolsäureester;
13) Polycarbonate und Polyestercarbonate;
14) Polyketone;
15) Mischungen bzw. Legierungen von o.g. Polymeren z.B. PP/EPDM, PA/EPDM oder ABS, PVC/EVA, PVC/ABS, PBC/MBS, PC/ABS, PBTP/ABS, PC/AS, PC/PBT, PVC/CPE, PVC/Acrylat, POM/thermoplastisches PUR, PC/thermoplastisches PUR, POM/Acrylat, POM/MBS, PPO/HIPS, PPO/PA6.6 und Copolymere, PA/HDPE, PA/PP, PA/PPO, PBT/PC/ABS oder PBT/PET/PC, sowie TPE-O, TPE-S und TPE-E;
16) Duroplaste wie PF, MF oder UF oder Mischungen davon;
17) Epoxidharze - Thermoplaste und Duroplaste;
18) Phenolharze;
19) Wood-Plastic-Composites (WPC) sowie Polymere auf PLA-, PHB- und Stärke-Basis.

Die Konzentration des mindestens einen beanspruchten Triazin-interkalierten Metall-Phosphates (a) und Komponente (b) in einem Polymer oder einer Polymermischung betragen vorzugsweise 0,1 bis 60 Gew.-% bezogen auf das zu verarbeitende Polymer.

Das durch Zusatz des mindestens einen erfindungsgemäßen Metall-Phosphats so flammgeschütze Material kann zu Fasern, Folien, Gussartikeln verarbeitet sowie zur Behandlung von Oberflächen verwendet werden.

Das mindestens eine erfindungsgemäße Metall-Phosphat kann auch zur Oberflächenbehandlung (Imprägnierung) von Fasern, Folien, Textilien oder andere technische Materialien verwendet werden.

Dementsprechend betrifft die vorliegende Erfindung die Verwendung einer erfindungsgemäßen Zusammensetzung in einem als Flammschutzmittel in einem Polymer, Papier, Textilien oder Wood Plastic Composites (WPC). Insbesondere handelt es sich bei dem Polymer um einen Thermoplasten, vorzugsweise ausgewählt aus der Gruppe bestehend aus Polyamid, Polycarbonat, Polyolefin, Polystyrol, Polyester, Polyvinylchlorid, Polyvinylalkohol, ABS und Polyurethan oder ein Duroplast ist, vorzugsweise ausgewählt aus der Gruppe bestehend aus Epoxidharz, Phenolharz und Melaminharz.

Die vorliegende Erfindung betrifft weiterhin die Verwendung der erfindungsgemäßen Triazin-interkalierten Metall -Phosphate mit offenen Gerüststrukturen für die Herstellung von Lacken, Klebstoffen, Gießharzen, Beschichtungen, Thixotropiemittel und Flammschutzmittel für Polymere.

Weiterhin betrifft die vorliegende Erfindung die Verwendung des mindestens einen erfindungsgemäßen Metall-Phosphats als Füllstoff in Polymeren.

Ein weiterer Gegenstand der vorliegenden Erfindung betrifft Verbindungen der allgemeinen Formel (I)

(A-H)ₐ⁽⁺⁾[M_{b}^{m+}(H₂PO₄)ₓ₁⁽⁻⁾(HPO₄)ₓ₂²⁽⁻⁾(PO₄)ₓ₃³⁽⁻⁾(PO₃)_{y}⁽⁻⁾]^{(a-)}*pH₂O (I)

wobei
(A - H)⁽⁺⁾ ein Triazin-Derivat der Formeln (II-1, II-2 oder II-3) 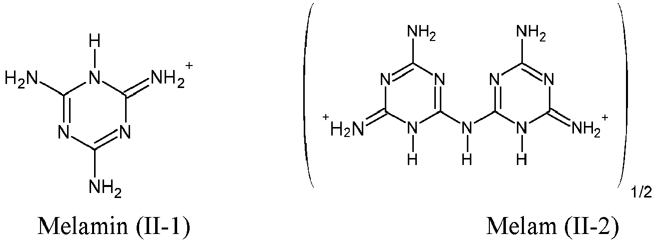 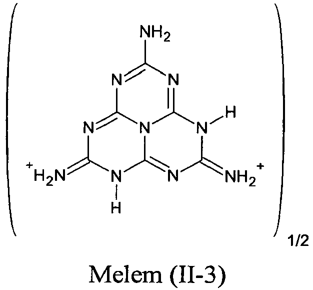
jedes **M** = Al ist,
**a** 2,
**b** 1**,**
**m** 3**,**
**x₁** = 0 oder 1, **x₂** = 0 oder 2, **x₃** = 1 oder 0, **y** = 2 oder 0 und p 0 bis 5 ist und wobei gilt: a + mb = x₁ + 2x₂ + 3x₃ +y.

Darüber hinaus betrifft die Erfindung ein Verfahren zur Herstellung der oben genannten Verbindungen den Schritt enthaltend
Umsetzen einer Verbindung (A), wobei (A) ein Triazin der Formeln (II-4), (II-5) oder (II-6) 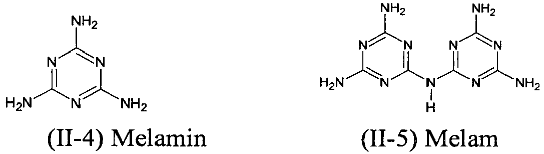 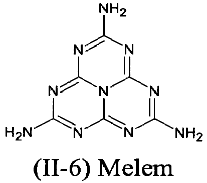 ist mit einem aziden Metall-Phosphat der Formel Hₐ⁽⁺⁾[M_{b}^{m+}(H₂PO₄)ₓ₁⁽⁻⁾(HPO₄)ₓ₂²⁽⁻⁾ (PO₄)ₓ₃³⁽⁻⁾ (PO₃)_{y}⁽⁽⁻⁾]^{(a-)}*pH₂O, wobei jedes M = Al.

In einem erfindungsgemäßen Verfahren zur Herstellung des erfindungsgemäßen Metall-Phosphats kann die Umsetzung in Wasser und vorzugsweise zwischen 20 und 90 °C, besonders bevorzugt zwischen 20 und 60 °C und ganz besonders bevorzugt zwischen 20 und 40 °C stattfinden.

Ein weiterer Gegenstand der vorliegenden Erfindung ist eine Verbindung erhältlich nach dem oben beschriebenen erfindungsgemäßen Verfahrens.

Insbesondere zeichnen sich solche Verbindungen dadurch aus, dass die Brutto-Zusammensetzung ein Melamin-Aluminiumphosphat [(Melamin-H)₂⁺ [AlP₃O₁₀]²⁽⁻⁾]_{z} ist, folgende ³¹P-MAS-NMR-Verschiebungen (δ-Werte) aufweist: -10,6ppm, -22,0ppm, - 24,5ppm und -27,6ppm und im ²⁷Al-NMR-Spektrum eine einzige Verschiebung um 40ppm zeigt. Insbesondere ist die Brutto-Zusammensetzung ein Melamin-Zinkphosphat [(Melamin-H)₂⁺ [ZnP₂O₇]²⁽⁻⁾]_{z} mit folgenden ³¹P-MAS-NMR-Verschiebungen (δ-Werte): +6,2ppm, +3,7ppm, +2,0ppm, -2,5ppm, -5,5ppm, -8,2ppm, -10,7ppm, -12,1ppm, -22,2ppm und -24,7ppm.

Das jeweilige Metall-Phosphat kann z.B. durch Vormischung in Form von Pulver und/oder Granulat in einem Mixer und dann durch Homogenisieren in einer Polymerschmelze durch Compoundieren (u.a. in einem Doppelschneckenextruder) hergestellt werden. Das Metall-Phosphat kann evtl. auch bei der Verarbeitung direkt zugegeben werden.

Als Metall -Phosphate für die Herstellung von Triazin-interkalierten Metall-Phosphaten mit offenen Gerüststrukturen kommen insbesondere Schicht-Phosphate der Formeln M(H₂PO₄)₃ und M(H₂PO₄)₂ (M = Al) und
kondensierte Phosphate wie Triphosphate bzw. Pyrophosphate der Formeln H₂AlP₃O₁₀ in Frage.

Am besten werden die Systeme jedoch über eine Reaktion mit Melamin als Templat in wässriger acider Metallsalzlösung hergestellt. Ein Alternativ-Verfahren besteht in der Umsetzung von Triazinphosphaten mit wässrigen Metallsalz-Lösungen (in Anlehnung an Angew. Chem., 1999, 111, 3688-3692).

Die so hergestellten Metall-Phosphate mit offenen Gerüststrukturen weisen Ortho-Phosphat (HₓPO₄-Typ mit x = 2, 1 oder 0), Pyrophosphat oder Triphosphat als Komplexliganden auf, wobei zwischen die Gitterschichten oder in die Hohlräume Melamin in protonierter Form (Melamin-Kation) interkaliert wird und bei Schichtstrukturen eine Aufweitung der Schichtabstände stattfindet.

In der weiteren Verarbeitung werden die erfindungsgemäßen Triazin-interkalierten Metall-Phosphate in eine geeignete Polymermatrix eingearbeitet. Geeignete Polymere, welche als Substrat verwendet werden können, sind an sich bekannt. Für die Einarbeitung bevorzugt sind thermoplastische Polymere und duroplastische Polymersysteme, Kautschuke und Textilien.

Melamin ist als Interkalat bevorzugt.

Mit Ortho-Phosphat als Liganden lassen sich die neuen Interkalate beispielhaft wie folgt darstellen, wobei **(A** - **H)⁽⁺⁾** (Mel-H)⁽⁺⁾ (Melamin-Kation) ist:
1. (Mel-H)⁽⁺⁾[Al³⁽⁺⁾(HPO₄)₂²⁽⁻⁾]⁽⁻⁾
2. (Mel-H)⁽⁺⁾[Al³⁽⁺⁾(HPO₄)₂²⁽⁻⁾H₂O]⁽⁻⁾
3. (Mel-H)₂⁽⁺⁾[Al³⁽⁺⁾(H₂PO₄)⁽⁻⁾(HPO₄)₂²⁽⁻⁾]²⁽⁻⁾
4. (Mel-H)₂⁽⁺⁾[Al³⁽⁺⁾(PO₄)³⁽⁻⁾(PO₃)₂⁽⁻⁾]²⁽⁻⁾ (Triphosphat-Typ)
wobei Aquo(Komplex)-Wasser durch thermische Behandlung entfernt werden kann.

Besonders bevorzugt sind 3 und 4.

Ein weiterer Gegenstand der vorliegenden Erfindung ist ein Verfahren zur Herstellung eines flammgeschützten, verformbaren Polymers, wobei das mindestens eine erfindungsgemäße, Triazin-interkalierte Metall-Phosphat in dem Polymer exfoliert wird.

Ein weiterer Gegenstand der Erfindung ist die Erzielung von antikorrosiven Schutzeffektes durch Coating von Metalloberflächen.

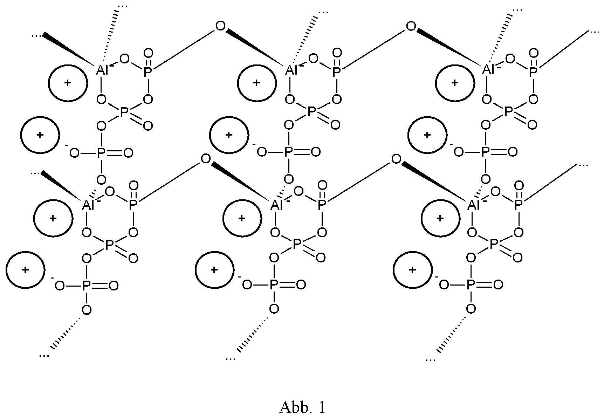

Abb. 1 zeigt beispielhaft einen Gitterausschnitt aus einem Interkalationsmodel von Melamin in Aluminiumtriphosphat (AlH₂P₃O₁₀)-Schichten (⊕ = Melaminium-Kation).

Die Erfindung wird durch nachfolgende Beispiele näher erläutert.

Eingesetzte Substanzen: Melamin (DSM); Aluminium-tris-dihydrogenphosphat (50%ige Lösung in Wasser) (PRAYON Deutschland), Zinkoxid, ortho-Phosphorsäure (ALDRICH)

### Beispiel 1: Synthese von Bis-Melamin-alumo-dihydrögenphosphat-bis hydrogenphosphat

### (Produkt A) -Precursor-Verbindung

(C₃H₇N₆)₂⁽⁺⁾[Al(H₂PO₄)(HPO₄)₂]²⁽⁻⁾

**(a** = 2, **M** = Al, **b** = 1, **m** = 3, **x₁** = 1, **x₂** = 2, **x₃** = 0, **y** = 0, **p** = 0)

100,9 g (0,8 Mol) Melamin werden in 2,4 l Wasser unter Rühren in der Wärme (40 bis 60 °C) gelöst. In dieser Lösung tropft man 254,4 g (0,4 Mol) Aluminium-tris-dihydrogenphosphat (50%ige Lösung in Wasser), wobei sich ein dicker Brei bildet. Anschließend wird 30 Min. nachgerührt, auf Raumtemperatur abgekühlt, der entstandene weiße Niederschlag abgesaugt, mit Wasser nachgewaschen und bei 120 °C gewichtskonstant getrocknet.
Ausbeute: 211,7 g entspricht 92,8 % d.Th.
Elementar-Analyse: C: **12,7** % (12,6 %); H: **3,3%** (3,2 %); N: 29,9% (29,5 %); Al: **4,7%**
(4,7 %); P: **16,4** % (16,3 %) (Theorie-Werte)

### Beispiel 2: Synthese von Bis-Melamin-alumo-triphosphat

### (Produkt B)

(C₃H₇N₆)₂⁽⁺⁾[Al³⁽⁺⁾(PO₄)³⁽⁻⁾(PO₃)₂⁽⁻⁾]²⁽⁻⁾

**(a** = 2, **M** = Al, **b** = 1, **m** = 3, x₁ = 0, x₂ = 0, **x₃** = 1, **y** = 2, **p** = 0)

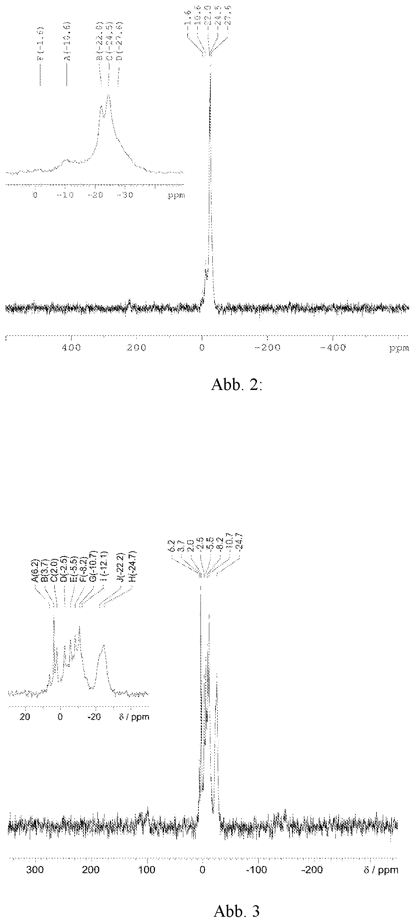

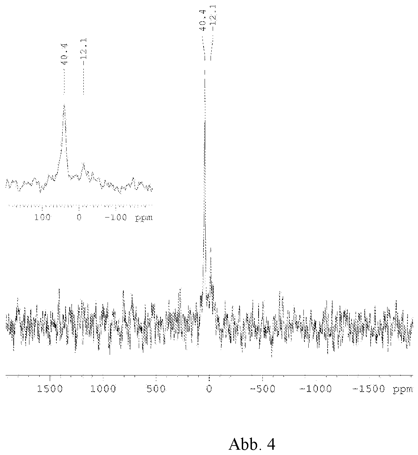

**Produkt (A)** wird unter öfterem Mischen 5 h bei 280 °C nahezu gewichtskonstant getempert. Das resultierende weiße Produkt besitzt folgende Zusammensetzung:
Elementar-Analyse: C: **13,5** % (13,5 %); H: **2,6** % (2,6 %); N: **30,1** % (31,5); Al: **5,1** % (5,1 %); P: **17,5** % (17,4 %) (Theorie-Werte)
³¹P-MAS-NMR-Verschiebungen (δ-Werte): -10,6ppm, -22,0ppm, -24,5ppm und -27,6ppm (siehe Abb. 2). Hierbei zeigt Abb. 2 das quantitatives ³¹P-NMR Spektrum von Bis-Melamin-alumo-triphosphat (Produkt B) (v_{MAS}=20KHz, ¹H-entkoppelt)).
²⁷Al-NMR-Spektrum: einzige Verschiebung um 40ppm (s. Abb. 4, v_{MAS}=20KHz).

### Vergleichsbeispiel 3: Synthese von Tris-Melamin-alumo-tris-hydrogenphosphatdihydrat

### (Produkt C)-Precursor-Verbindung

(C₃H₇N₆)₃⁽⁺⁾[Al(HPO₄)₃]³⁽⁻⁾*2H₂O

**(a** = 3, **M** = Al, **b** = 1, **m=** 3, **x₁** = 0, **x₂** = 3, **x₃** = 0, **y** = 0, **p** =2).

94,6 g (0,75 Mol) Melamin werden in 2,3 l Wasser unter Rühren in der Hitze gelöst. In diese Lösung tropft man 159,0 g (0,25 Mol) Aluminium-tris-dihydrogenphosphat (50%ige Lösung in Wasser), wobei sich ein voluminöser Brei bildet. Anschließend wird 30 Minuten nachgerührt, auf Raumtemperatur abgekühlt, der entstandene weiße Niederschlag abgesaugt, zweimal mit Wasser nachgewaschen und bei 120 °C gewichtskonstant getrocknet. Ausbeute: 174,0 g entspr. 95,0 % d.Th.
Elementar-Analyse: C: **14,8** % (14,8 %); H: **3,5** % (3,9 %); N: 33**,8** % (34,4 %) (Theorie-Werte)

### Beispiel 4: Synthese von Produkt B ausgehend von Produkt C.

Herstellung von **Produkt C** wie in Beispiel 3, aber mit nachfolgender Temperung 5 h bei 210 °C. Es resultiert als Vorstufe Tris-melamin-alumo-tris-dihydrogenphosphatmonohydrat.
(C₃H₇N₆)₃⁽⁺⁾[Al(HPO₄)₃]³⁽⁻⁾*H₂O.
Ausbeute: 165,7 g entspr. 92,8 % d. Th.
Elementar-Analyse: C: **15,1** % (15,1 %); H: **4,3** % (3,7 %); N: **35,1** % (35,3 %); (Theorie-Werte)

Aus dieser Vorstufe wird durch erneutes Tempern bei 280 °C, 6 h **Produkt B** erhalten, wobei eine Gewichtsabnahme von 25 % stattfindet. Es resultiert Bis-melamin-alumo-triphosphat
(C₃H₇N₆)₂⁽⁺⁾[Al³⁽⁺⁾(PO₄)³⁽⁻⁾(PO₃)₂⁽⁻⁾]²⁽⁻⁾ (Ausbeute quant.)
Elementar-Analyse: **C: 13,4** % (13,5 %); **H: 4,0** % (2,6 %); **N: 29,7** % (31,5 %); (Theorie-Werte)

Hieraus ist ersichtlich, dass zu **Produkt B** durch Verwendung von **Produkt C** auch ein Alternativ-Zugang möglich ist. Dieses Verfahren ist jedoch in der Praxis unwirtschaftlich, da ca. ein Drittel des eingesetzten Melamins durch Temperung wieder entfernt werden muss.

Verzichtet man jedoch auf die Temperung, so ist eine Einarbeitung in Polyamide, Polycarbonate und Polyester stark erschwert, da nennenswerte Mengen an Melamin absublimieren. Bei Einsatz des nach Bespiel 2 hergestellten Produktes B treten diese Schwierigkeiten jedoch nicht auf.

### Beispiel 5: Synthese von Bis-Melamin-zinko-diphosphat

### (Produkt D)

(C₃H₇N₆)₂⁽⁺⁾[Zn²⁽⁺⁾(PO₄)³⁽⁻⁾(PO₃)⁽⁻⁾]²⁽⁻⁾

**(a** = 2, **M** = Zn, **b** = 1, **m=** 2, **x₁** = 0, **x₂** = 0, **x₃** = 1, **y** = 1, **p** =0).

Produkt D erhalten nach obiger Vorschrift, wird 5 h bei 280 °C getrocknet, wobei eine Gewichtsabnahme von ca. 6,0 % stattfindet.
Elementar-Analyse: C: **15,1** % (14,6 %); H: **2,8** % (2,9 %); N: **34,0** % (34,1 %); Zn: 12,6 % (13,3 %); P: 12,2 % (12,2 %). (Theorie-Werte)
³¹P-MAS-NMR-Verschiebungen (δ-Werte): +6,2ppm, +3,7ppm, +2,0ppm, -2,5ppm, - 5,5ppm, -8,2ppm, -10,7ppm, -12,1ppm, -22,2ppm und -24,7ppm. (s. Abb. 3). Hierbei zeigt Abb. 3 das quantitative ³¹P-NMR Spektrum von Bis-Melamin-zinko-diphosphat (Produkt D) (v_{MAS}=20KHz).

### Beispiel 6: Statische thermische Behandlung der Precursor-Produkten A und C:

Die Ergebnisse sind in der Tab. 1. zusammengefasst.

**Tabelle 1: Thermische Behandlung von Precursor-Produkten**

| | Produkt A (%) | Produkt C (%) |
|---|---|---|
| | 100 | 100 |
| 200 °C / 2h | 94,9 | 93,9 |
| 240 °C / 2h | 91,4 | 86,0 |
| 280 °C / 2h | 89,5 | 82,4 |
| 300 °C /2h | 85,9 | 77,7 |
| 300 °C/ 4h | 82,7 | 76,0 |

Wie aus Tabelle 1 ersichtlich ist, ist das erfindungsgemäßen Produkt **A** wesentlich thermostabiler als der Stand der Technik **Produkt C** (WO-A 2009/015772). Dieses Verhalten war überraschend, da nicht vorhersehbar.

### Beispiel 7: Statische thermische Behandlung der Temper-Produkte B, D und MPP (Melaminpolyphosphat, Stand der Technik)

Die Ergebnisse sind in der Tab. 2. zusammengefasst.

**Tabelle 2: Thermische Behandlung von Temper-Produkten**

| | Produkt B(%) | Produkt D (%) | Produkt MPP (%) |
|---|---|---|---|
| | 100 | 100 | 100 |
| 200 °C / 2h | 99,8 | 99,2 | 99,1 |
| 240 °C / 2h | 99,5 | 98,9 | 98,9 |
| 280 °C / 2h | 98,3 | 97,4 | 98,0 |
| 300 °C /2h | 94,3 | 94,1 | 91,3 |
| 300 °C/ 4h | 89,7 | 92,7 | 83,7 |

Wie aus Tabelle 2 ersichtlich ist, sind die erfindungsgemäßen **Produkte B** und **D** wesentlich thermostabiler als der Stand der Technik **MPP.** Dieses Verhalten war überraschend, da nicht vorhersehbar.

### Anwendungstechnische Prüfung in PVC

### I. Herstellung der Walzfelle:

Die nach Tabelle 1 **(R-1, R-2)** zubereiteten Trockenmischungen werden auf einem Collin-Labormeßwalzwerk (Modell: W100E, BJ: 2005, Fa. COLLIN) jeweils 5 Minuten (Walzendurchmesser: 110 mm, 15 UpM, Friktion: -15 %) bei der angegebenen Temperatur plastifiziert. Die so erhaltenen Folien (Dicke 0,3mm) werden weiteren Messungen zugeführt.

### II. Durchführung des statischen Hitzetestes (SHT):

Von den nach I hergestellten Walzfellen werden Teststreifen (15 mm x 15 mm) ausgeschnitten. Diese werden in einem METRASTAT-Testofen IR 700 (DR. STAPFER GmbH, Düsseldorf) bei der angegebenen Temperatur bis zur signifikanten Verfärbung belastet. Im Anschluss wird der YI-Wert (Yellowness-Index) nach DIN 53381 mit einem Spectro-Guide Farbmessgerät (Fa. BYK-GARDNER) bestimmt und mit dem YI-Wert des unbelasteten Walzfelles verglichen (Nullminutenwert). Die Ergebnisse sind tabellarisch zusammengefasst. Es gilt, je kleiner der YI-Wert zu einem bestimmten Zeitpunkt, desto besser ist das Farbverhalten.

### III. Durchführung der Flammschutzprüfung:

Die oben hergestellten Walzfelle werden zu Pressplatten (120x100x3mm) verarbeitet und einer Flammschutzprüfung in Anlehnung an UL94 zugeführt. Der UL94-Test ist in *"*Flammability of Plastic Materials for Parts in Devices and Appliances", 5th edition, October, 1996 beschrieben.

### IV. Bestimmung der mechanischen Eigenschaften:

Die mechanischen Eigenschaften wurden mittels *Instron 5569* (5kN side action grips) nach ASTM D412 bestimmt.

### V. Durchführung der NMR Messungen:

Alle Messungen wurden auf einem Bruker Avance-II 200 Festkörper-MAS-Spektrometer mit 4.7 T Magnet und einem Doppelresonanzprobenkopf für 2.5 mm Rotoren unter *magic angle spinning* (MAS) Bedingungen durchgeführt. Die verwendeten Rotationsfrequenzen v*_{MAS}* sind bei den entsprechenden Messungen angegeben. Chemische Verschiebungen sind relativ zu den aktuell von der IUPAC empfohlenen Referenzsubstanzen angegeben (²⁷Al: 1.1 M Al(NO₃)₃ in D₂O; ³¹P: 85%ige Phosphorsäure), wobei die Spektrometerkalibrierung mit Hilfe der vereinheitlichten Verschiebungsskala auf die Protonenresonanz von TMS vorgenommen wurde.

### Es wurden folgende Formulierungen geprüft:

### Beispiel 8: Prüfung in Weich-PVC:

Folgende Trockenmischungen werden hergestellt (Tabelle 3)- Einwaage in Gew.-Teilen:

**Tab. 3: Formulierungen**

| Komponenten | **(R-1)** | **(R-2)** |
|---|---|---|
| PVC (Evipol SH 7020) K-Wert = 70 | 100 | 100 |
| Weichmacher (DINP)¹⁾ | 50 | 50 |
| Zinkstearat | 0,6 | 0,6 |
| Hydrotalcit²⁾ | 2,9 | 2,9 |
| Antioxidans (Bisphenol A) | 0,5 | 0,5 |
| Flammschtzmittel 1 (ATH)³⁾ | 60 | 25 |
| Flammschutzmittel 2 (Produkt B) | -- | 5 |
| Flammschutzwirkung (Brenndauer in Sek. nach 3 Zündungen) | 0/1/1 | 0/1/1 |

| | | |
|---|---|---|
| ¹⁾ Diisononylphthalat, ex BASF ²⁾ Sorbacid 911, ex SÜD CHEMIE ³⁾ Aluminiumtrihydroxid, APYRAL 40CD, ex NABALTEC | | |

Wie aus Tabelle 3 ersichtlich, schneidet die erfindungsgemäße Formulierung (R-2) vergleichbar gut ab, wie der Stand der Technik Beispiel **(R-1)**

**Tabelle 4: SHT (200 °C) nach II**

| Zeit [min] | **(R-1)** | **(R-2)** |
|---|---|---|
| 3 | 10,3 | 5,8 |
| 6 | 10,0 | 5,5 |
| 9 | 11,0 | 5,7 |
| 12 | 11,5 | 6,4 |
| 15 | 12,9 | 7,4 |
| 18 | 13,9 | 8,6 |
| 21 | 15,5 | 10,3 |
| 24 | 18,8 | 12,7 |
| 27 | 22,0 | 15,8 |
| 30 | 25,8 | 19,3 |
| 33 | 31,7 | 24,9 |
| 36 | 40,3 | 33,3 |
| 39 | 52,9 | 44,5 |
| 42 | 70,5 | 62,7 |
| 45 | 95,3 | 76,7 |
| 48 | 110,8 | 85,5 |
| 51 | 117,2 | 89,2 |
| 54 | 118,4 | 90,9 |
| 57 | 117,6 | 91,3 |
| 60 | 115,72 | 92,4 |

Wie aus der Tabelle 4 ersichtlich, zeigt die erfindungsgemäße Formulierung **(R-2)** ein signifikant besseres Farbverhalten, insbesondere in Bezug auf die Anfangsfarbe als die nicht erfindungsgemäße Formulierung **(R-1)**

**Tab. 5.: Mechanische Eigenschaften**

| | Zugfestigkeit [MPa] | Bruchdehnung [%] | Young Modulus [MPa] |
|---|---|---|---|
| **(R-1)** | 13,64 | 331,38 | 33,59 |
| **(R-2)** | 15,24 | 368,45 | 25,27 |

Tabelle 5 zeigt, dass die mechanischen Eigenschaften der erfindungsgemäßen Formulierung **(R-2)** im Vergleich zum Stand der Technik **(R-1)** sogar noch verbessert werden.

## Patentansprüche

1. Verbindungen der allgemeinen Formel (I)
(A-H)ₐ⁽⁺⁾[M_{b}^{m+}(H₂P0₄)ₓ₁⁽⁻⁾(HP0₄)ₓ₂²⁽⁻⁾(PO₄)ₓ₃³⁽⁻⁾(PO₃)_{y}⁽⁻⁾]^{(a-)}*pH₂O (I)
wobei
(A - H)⁽⁺⁾ ein Triazin-Derivat der Formeln (II- 1 , II-2 oder II-3) 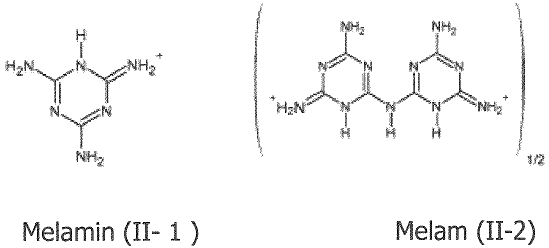 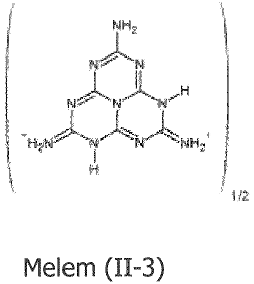
jedes **M** = Al ist,
**a** ist 2,
**b ist** 1,
**m** = 3,
und
**x₁** = 0 oder 1; **x₂** = 0 oder 2; **x₃** = 1 oder 0; **y** = 2 oder 0 und p = 0 bis 5, wobei: a + mb = x₁ + 2x₂ + 3x₃ + y.

2. Flammschutzmittelzusammensetzung enthaltend
(a) mindestens eine Verbindung gemäß Anspruch 1 als ein Triazin-interkaliertes Metall-Phosphat mit mindestens einer Monomereinheit der allgemeinen Formel (I), und
(b) mindestens eine weitere von (a) verschiedene Flammschutzmittelkomponente.

3. Zusammensetzung nach Anspruch 2, **dadurch gekennzeichnet, dass** diese mindestens eine weitere Komponente (b) eine Metallverbindung, die kein Metall-Phosphat der Komponente (a) ist, oder/und mindestens eine metallfreie Phosphorverbindung ist.

4. Zusammensetzung nach Anspruch 3, **dadurch gekennzeichnet, dass** die mindestens eine Metallverbindung (b) ein Metalloxid, ein Metallhydroxid, ein Metallphosphat, ein Metallpyrophosphat, ein Hydrotalcit, ein kationisch- oder anionisch-modifizierter Organoclay, ein Stannat oder Molybdat-Salz, ein Metallborat oder Metall-Phosphinat der Formel (III) ist: 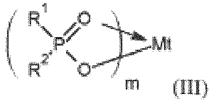 wobei R¹ und R² Wasserstoff oder ein geradkettiger oder ein verzweigter C₁ - C₆-Alkylrest oder ein Phenylrest; und Mt = Ca, Mg, Zn oder AI und m = 2 oder 3 ist.

5. Zusammensetzung nach Anspruch 3 oder 4, **dadurch gekennzeichnet, dass** die mindestens eine Metallverbindung (b) Diantimontrioxid,
Diantimontetroxid, Diantimonpentoxid oder Zinkoxid ist.

6. Zusammensetzung nach Anspruch 3 oder 4, **dadurch gekennzeichnet, dass** die mindestens eine Metallverbindung (b) Magnesiumhydroxid (Brucit), Aluminiumtrihydroxid (ATH, Gibbsit) oder Aluminiummonohydroxid (Boehmit) ist.

7. Zusammensetzung nach Anspruch 3 oder 4, **dadurch gekennzeichnet, dass** die mindestens eine Metallverbindung (b) Magnesium- Aluminium-Hydroxo- Carbonat oder Calcium- Aluminium-Hydroxo-Carbonat ist.

8. Zusammensetzung nach Anspruch 3 oder 4, **dadurch gekennzeichnet, dass** die mindestens eine Metallverbindung (b) ein alkylsulfat- oder fettsäurecarboxylat- modifizierter Hydrotalcit oder ein langkettig quaternär - ammonium-modifiziertes Tonmineral ist.

9. Zusammensetzung nach Anspruch 3 oder 4, **dadurch gekennzeichnet, dass** die mindestens eine Metallverbindung (b) Ammoniumheptamolybdat, Ammoniumoctamolybdat, Zinkstannat oder Zinkhydroxystannat ist.

10. Zusammensetzung nach Anspruch 3 oder 4, **dadurch gekennzeichnet, dass** die mindestens eine Metallverbindung (b) ein Alkali-, Erdalkali- oder Zinkborat ist.

11. Zusammensetzung nach Anspruch 3 oder 4, **dadurch gekennzeichnet, dass** die mindestens eine Metallverbindung (b) Ca-, Mg-, Zn- oder Al-Phosphinat (Hypophosphit), Ca-, Mg-, Zn- oder Al-Phenyl(benzol)phosphinat oder Ca-, Mg-, Zn- oder A1-diethyl(ethan)phosphinat ist.

12. Zusammensetzung nach einem der Ansprüche 3 bis 11, **dadurch gekennzeichnet, dass** die mindestens eine metallfreie Phosphorverbindung (b) roter Phosphor, ein oligomerer Phosphatester, ein oligomerer Phosphonatester, ein zyklischer Phosphonatester, ein Thiopyrophosphorsäureester, ein Melaminpolyphosphat oder Ammoniumpolyphosphat, ein Hydroxyalkyl-Phosphinoxid, ein Tetrakis- hydroxyalkyl-Phosphoniumsalz, ein Phospholan(oxid)-Derivat oder Dihydrophopshol(oxid)-derivat, oder ein Phosphonitester, ist.

13. Zusammensetzung nach einem der Ansprüche 3 bis 12, **dadurch gekennzeichnet, dass** die mindestens eine metallfreie Phosphorverbindung ein oligomerer Phosphatester der Formel (IV) oder Formel (V) ist: 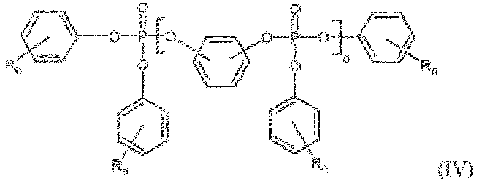 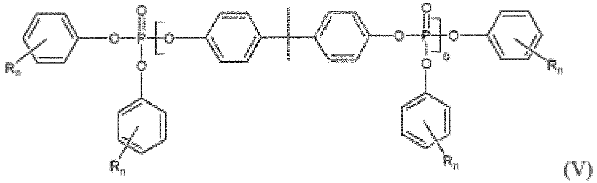 wobei jedes R jeweils unabhängig Wasserstoff, C₁ - C₄Alkyl oder Hydroxy, n = 1 bis 3 und o 1 bis 10 ist.

14. Zusammensetzung nach einem der Ansprüche 3 bis 12, **dadurch gekennzeichnet, dass** die mindestens eine metallfreie Phosphorverbindung ein oligomerer Phosphonatester der Formel (VI) ist: 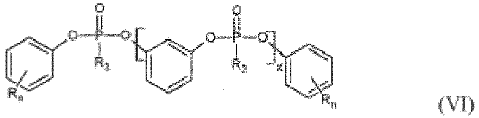 wobei R³ = Methyl oder Phenyl und x 1 bis 20 ist und R, n die Bedeutung wie in Anspruch 13 aufweisen.

15. Zusammensetzung nach einem der Ansprüche 3 bis 12, **dadurch gekennzeichnet, dass** die mindestens eine metallfreie Phosphorverbindung ein zyklischer Phosphonatester der Formel (VII) ist: 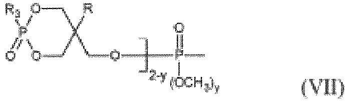 wobei y = 0 oder 2 ist.

16. Zusammensetzung nach einem der Ansprüche 3 bis 12, **dadurch gekennzeichnet, dass** die mindestens eine metallfreie Phosphorverbindung ein Thiopyrophosphorsäureester der Formel (VIII) ist:

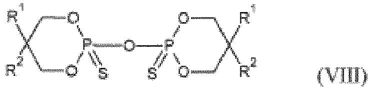

17. Zusammensetzung nach einem der Ansprüche 3 bis 12, **dadurch gekennzeichnet, dass** die mindestens eine metallfreie Phosphorverbindung als Phosphonitester in Form eines Benzolmonophenylesterderivat oder als ein DOPO-Derivat (9, 10-Dihydro-9-0xa-10-Phosphaphenanthren-10-oxid oder 6H-Dibenzo(c,e) (1,2)-oxaphosphorin-6-on)-Derivat der Formel (IX) oder (X) vorliegt:

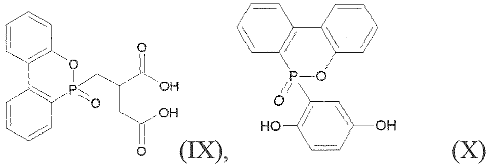

18. Zusammensetzung nach einem der Ansprüche 3 bis 17, **dadurch gekennzeichnet, dass** zusätzlich Polyole und/oder Aminouracile und/oder PO SS- Verbindungen und/oder Trishydroxyethylisocyanurat und/oder Melamincyanurat und/oder Blähgraphit enthalten sind.

19. Zusammensetzung nach Anspruch 18, **dadurch gekennzeichnet, dass** das Polyol Pentaerythrit, Dipentaerythrit oder Tripentaerythrit, das Aminouracil 1,3-Dimethyl-6-aminouracil und die POSS-Verbindung Methylsiloxan-basiert ist.

20. Zusammensetzung nach einem der Ansprüche 2 bis 19, **dadurch gekennzeichnet, dass** die Konzentration des mindestens ein Triazin-interkalierten Metall-Phosphates (a) und der Komponente (b) in einem Polymer oder einer Polymermischung vorzugsweise 0,1 bis 60 Gew.-% bezogen auf das zu verarbeitende Polymer betragen.

21. Verwendung einer Zusammensetzung nach einem der Ansprüche 2 bis 20 als Flammschutzmittel in einem Polymer, Papier, Textilien oder Wood Plastic Composites (WPC).

22. Verwendung nach Anspruch 21, **dadurch gekennzeichnet, dass** das Polymer ein Thermoplast ist, vorzugsweise ausgewählt aus der Gruppe bestehend aus Polyamid, Polycarbonat, Polyolefin, Polystyrol, Polyester, Polyvinylchlorid, Polyvinylalkohol, ABS und Polyurethan oder ein Duroplast ist, vorzugsweise ausgewählt aus der Gruppe bestehend aus Epoxidharz, Phenolharz und Melaminharz.

23. Verfahren zur Herstellung einer Verbindung nach Anspruch 1, den Schritt enthaltend Umsetzen einer Verbindung (A), wobei (A) ein Triazin der Formeln II-4), (II-5) oder (II-6) 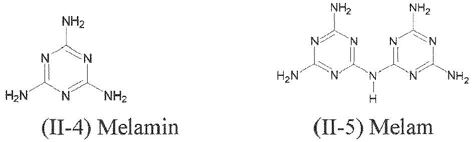 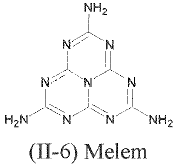 ist mit einem aziden Metall-Phosphat der Formel Hₐ⁽⁺⁾[M_{b}^{m+}(H₂P0₄)ₓ₁⁽⁻⁾(HP0₄)ₓ₂²⁽⁻⁾ (P0₄)ₓ₃³⁽⁻⁾(P0₃)_{y}⁽⁽⁻⁾]^{(a-)}*pH₂0, wobei jedes M = Al.

24. Verfahren nach Anspruch 23, **dadurch gekennzeichnet, dass** die Umsetzung in Wasser und vorzugsweise zwischen 20 und 90 °C, besonders bevorzugt zwischen 20 und 60 °C und ganz besonders bevorzugt zwischen 20 und 40 °C stattfindet.

## Claims

1. Compounds of general formula (I)
(A-H)ₐ⁽⁺⁾[M_{b}^{m+}(H₂PO₄)ₓ₁⁽⁻⁾(HPO₄)ₓ₂²⁽⁻⁾(PO₄)ₓ₃³⁽⁻⁾(PO₃)_{y}⁽⁻⁾]^{(a-)}* p H₂O (I)
where
(A - H)⁽⁺⁾ is a triazine derivative of formulas (II-1, II-2, or II-3) 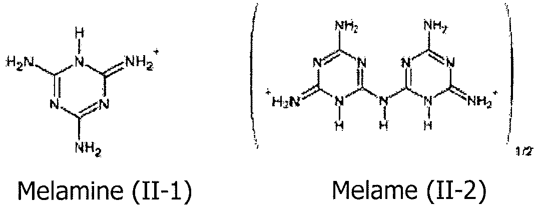 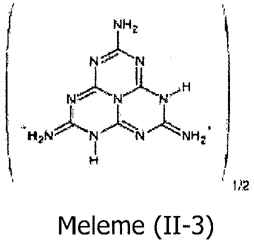 and
each M is Al,
a is 2,
b is 1,
m is 3,
and
x₁ = 0 or 1; x₂ = 0 or 2; x₃ = 1 or 0; y = 2 or 0, and p = 0 to 5,
where: a + mb = x₁ + 2x₂ + 3x₃ + y.

2. A flame retardant composition containing
(a) at least one compound according to Claim 1 as a triazine-intercalated metal phosphate having at least one monomer unit of general formula (I), and
(b) at least one further flame retardant component that is different from (a).

3. The composition according to Claim 2, **characterized in that** this at least one further component (b) is a metal compound that is not a metal phosphate of component (a), and/or is at least one metal-free phosphorus compound.

4. The composition according to Claim 3, **characterized in that** the at least one metal compound (b) is a metal oxide, a metal hydroxide, a metal phosphate, a metal pyrophosphate, a hydrotalcite, a cationically or anionically modified organoclay, a stannate salt or molybdate salt, a metal borate, or a metal phosphinate of formula (III) : 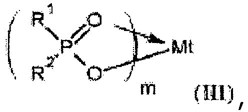 where R¹ and R² are hydrogen or a straight-chain or branched C₁-C₆ alkyl moiety or a phenyl moiety; and Mt = Ca, Mg, Zn, or Al and m = 2 or 3.

5. The composition according to Claim 3 or 4, **characterized in that** the at least one metal compound (b) is diantimony trioxide, diantimony tetroxide, diantimony pentoxide, or zinc oxide.

6. The composition according to Claim 3 or 4, **characterized in that** the at least one metal compound (b) is magnesium hydroxide (brucite), aluminum trihydroxide (ATH, gibbsite), or aluminum monohydroxide (boehmite).

7. The composition according to Claim 3 or 4, **characterized in that** the at least one metal compound (b) is magnesium aluminum hydroxocarbonate or calcium aluminum hydroxocarbonate.

8. The composition according to Claim 3 or 4, **characterized in that** the at least one metal compound (b) is an alkyl sulfate-modified or fatty acid carboxylate-modified hydrotalcite or a long-chain quaternary ammonium-modified clay mineral.

9. The composition according to Claim 3 or 4, **characterized in that** the at least one metal compound (b) is ammonium heptamolybdate, ammonium octamolybdate, zinc stannate, or zinc hydroxystannate.

10. The composition according to Claim 3 or 4, **characterized in that** the at least one metal compound (b) is an alkali borate, an alkaline earth borate, or zinc borate.

11. The composition according to Claim 3 or 4, **characterized in that** the at least one metal compound (b) is Ca, Mg, Zn, or Al phosphinate (hypophosphite), Ca, Mg, Zn, or Al phenyl(benzene)phosphinate, or Ca, Mg, Zn, or Al diethyl(ethane)phosphinate.

12. The composition according to one of Claims 3 to 11, **characterized in that** the at least one metal-free phosphorus compound (b) is red phosphorus, an oligomeric phosphate ester, an oligomeric phosphonate ester, a cyclic phosphonate ester, a thiopyrophosphoric acid ester, a melamine polyphosphate or ammonium polyphosphate, a hydroxyalkyl phosphine oxide, a tetrakis-hydroxyalkylphosphonium salt, a phospholane (oxide) derivative or dihydrophosphole (oxide) derivative, or a phosphonite ester.

13. The composition according to one of Claims 3 to 12, **characterized in that** the at least one metal-free phosphorus compound is an oligomeric phosphate ester of formula (IV) or formula (V): 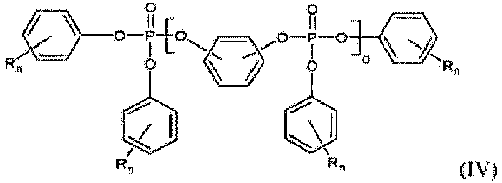 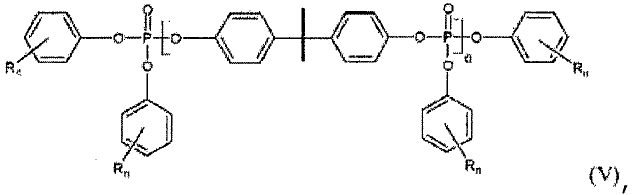 where each R is independently hydrogen, C₁-C₄ alkyl, or hydroxy, n = 1 to 3, and o = 1 to 10.

14. The composition according to one of Claims 3 to 12, **characterized in that** the at least one metal-free phosphorus compound is an oligomeric phosphonate ester of formula (VI): 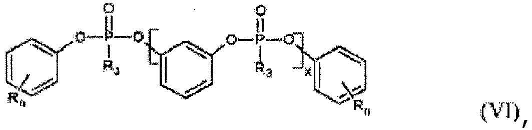 where R³ = methyl or phenyl and x = 1 to 20, and R, n have the meanings as in Claim 13.

15. The composition according to one of Claims 3 to 12, **characterized in that** the at least one metal-free phosphorus compound is a cyclic phosphonate ester of formula (VII): 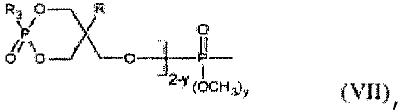 where y = 0 or 2.

16. The composition according to one of Claims 3 to 12, **characterized in that** the at least one metal-free phosphorus compound is a thiopyrophosphoric acid ester of formula (VIII):

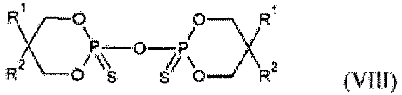

17. The composition according to one of Claims 3 to 12, **characterized in that** the at least one metal-free phosphorus compound is present as a phosphonite ester in the form of a benzenemonophenyl ester derivative or as a 9,10-dihydro-9-oxa-10-phosphaphenanthrene-10-oxide (DOPO) derivative or a 6*H*-dibenzene(c,e)(1,2)-oxaphosphorin-6-one) derivative of formula (IX) or (X):

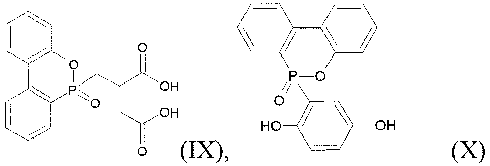

18. The composition according to one of Claims 3 to 17, **characterized in that** polyols and/or aminouracils and/or POSS compounds and/or tris-hydroxyethylisocyanurate and/or melamine cyanurate and/or expanded graphite are additionally contained.

19. The composition according to Claim 18, **characterized in that** the polyol is pentaerythrite, dipentaerythrite, or tripentaerythrite, the aminouracil is 1,3-dimethyl-6-aminouracil, and the POSS compound is based on methylsiloxane.

20. The composition according to one of Claims 2 to 19, **characterized in that** the concentration of the at least one triazine-intercalated metal phosphate (a) and component (b) in a polymer or a polymer mixture is preferably 0.1 to 60% by weight, based on the polymer to be processed.

21. Use of a composition according to one of Claims 2 to 20 as a flame retardant in a polymer, paper, textiles, or wood plastic composites (WPC).

22. Use according to Claim 21, **characterized in that** the polymer is a thermoplastic that is preferably selected from the group comprising polyamide, polycarbonate, polyolefin, polystyrene, polyester, polyvinyl chloride, polyvinyl alcohol, ABS, and polyurethane, or is a thermoset that is preferably selected from the group comprising epoxy resin, phenolic resin, and melamine resin.

23. A method for producing a compound according to Claim 1, containing the step of
reacting a compound (A), where (A) is a triazine of formulas (II-4), (II-5), or (II-6) 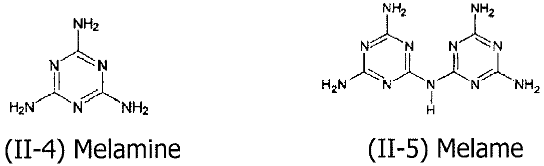 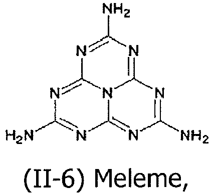 with an azide metal phosphate of formula Hₐ⁽⁺⁾[M_{b}^{m+}(H₂PO₄)ₓ₁⁽⁻⁾(HPO₄)ₓ₂²⁽⁻⁾ (PO₄)ₓ₃³⁽⁻⁾(PO₃)_{y}⁽⁻⁾]^{(a-)}* p H₂O, where each M = Al.

24. The method according to Claim 23, **characterized in that** the reaction takes place in water and preferably between 20 and 90°C, particularly preferably between 20 and 60°C, and very particularly preferably between 20 and 40°C.

## Revendications

1. Composés de la formule générale (I)
(A-H)ₐ⁽⁺⁾[M⁺_{b}^{m+}(H₂PO₄)ₓ₁⁽⁻⁾(HPO₄)ₓ₂²⁽⁻⁾(PO₄)ₓ₃³⁽⁻⁾(PO_{3)y}⁽⁻⁾]^{(a-)}*pH₂O (I)
où
(A - H)⁽⁺⁾ est un dérivé de la triazine des formules (II-1, II-2, ou II-3) 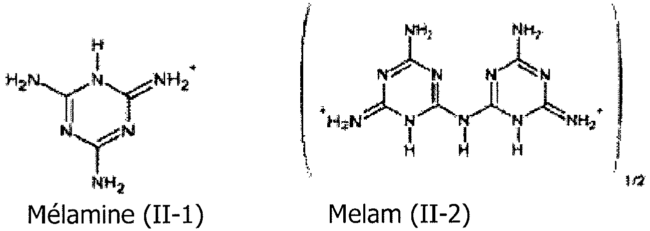 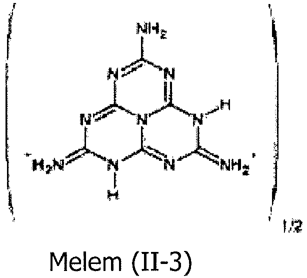 et
chaque M = al,
a est 2,
b est 1,
m = 3,
et
x₁ = 0 ou 1 ; x₂ = 0 ou 2 ; x₃ = 0 ou 1 ; y = 2 ou 0 et p = 0 à 5,
avec a + mb = x₁ + 2x + 3x₂ + y₃.

2. Composition retardateur de contenant
(a) au moins un composé selon la revendication 1, en tant que phosphate de métal à triazine intercalaire comportant au moins une unité monomère de la formule générale (I) et
(b) au moins un autre composant retardateur de flamme différent de (a).

3. Composition selon la revendication 2, **caractérisée en ce que** cet au moins un autre composant (b) est un composé métallique, qui n'est pas un phosphate métallique du composant (a), et/ou au moins un composé du phosphore exempt de métal.

4. Composition selon la revendication 3, **caractérisée en ce que** l'au moins un composé métallique (b) est un oxyde métallique, un hydroxide métallique, un phosphate métallique, un pyrophosphate métallique, un hydrotalcite, une argile organique à modification cationique ou anionique, un sel stannate ou molybdate, un borate métallique, ou un phosphinate métallique de la formule (III) : 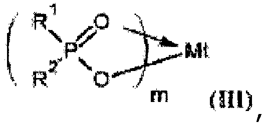 où R¹ et R² sont un atome d'hydrogène ou un radical alkyle en C₁ à C₆ linéaire ou ramifié ou un radical phényle ; et Mt = Ca, Mg, Zn, ou Al et m = 2 ou 3.

5. Composition selon la revendication 3 ou 4, **caractérisée en ce que** l'au moins un composé métallique (b) est du trioxyde de diantimoine, du tétroxyde de diantimoine, du pentoxyde de diantimoine, ou de l'oxyde de zinc.

6. Composition selon la revendication 3 ou 4, **caractérisée en ce que** l'au moins un composé métallique (b) est l'hydroxyde de magnésium (brucite), le trihydroxyde d'aluminium (ATH, gibbsite), ou le monohydroxyde d'aluminium (boehmite).

7. Composition selon la revendication 3 ou 4, **caractérisée en ce que** l'au moins un composé métallique (b) est le hydroxocarbonate de magnésium-aluminium ou l'hydroxocarbonate d'aluminium et de calcium.

8. Composition selon la revendication 3 ou 4, **caractérisée en ce que** l'au moins un composé métallique (b) est un hydrotalcite modifié par un sulfate d'alkyle ou un carboxylate d'acide gras ou un minéral argileux modifié par ammonium quaternaire à longue chaîne.

9. Composition selon la revendication 3 ou 4, **caractérisée en ce que** l'au moins un composé métallique (b) est un heptamolybdate d'ammonium, un octamolybdate d'ammonium, un stannate de zinc, ou un hydroxystannate de zinc.

10. Composition selon la revendication 3 ou 4, **caractérisé en ce que** le au moins un composé de métal (b) est un borate alcalin, un borate alcalino-terreux, ou borate de zinc.

11. Composition selon la revendication 3 ou 4, **caractérisée en ce que** l'au moins un composé métallique (b) est un phosphinate de Ca, Mg, Zn, ou Al (hypophosphite), un phényl(benzène)phosphinate de Ca, Mg, Zn, ou Al, ou un diéthyl(éthan)phosphinate de Ca, Mg, Zn, ou Al.

12. Composition selon l'une des revendications 3 à 11, **caractérisée en ce que** l'au moins un composé de phosphore exempt de métal (b) est du rouge phosphore rouge, un phosphate-ester oligomère,un phosphonate-ester oligomère, un phosphonate-ester cyclique, un ester d'acide thiopyrophosphorique, un polyphosphate de mélamine ou un polyphosphate d'ammonium, un phosphinoxyde d'hydroxyalkyle, un sel phosphonium de tétrakis-hydroxyalkyle, un dérivé de phospholan(oxyde) ou un dérivé de dihydrophosphol(oxyde), ou une ester de phosphonite.

13. Composition selon l'une des revendications 3 à 12, **caractérisée en ce que** l'au moins un composé de phosphore exempt de métal est un phosphate-ester oligomère de la formule (IV) ou la formule (V) : 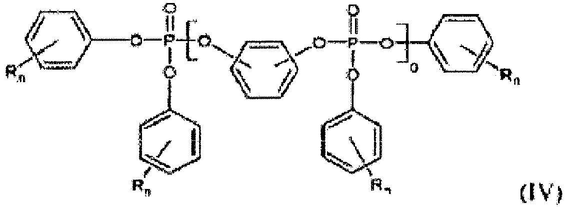 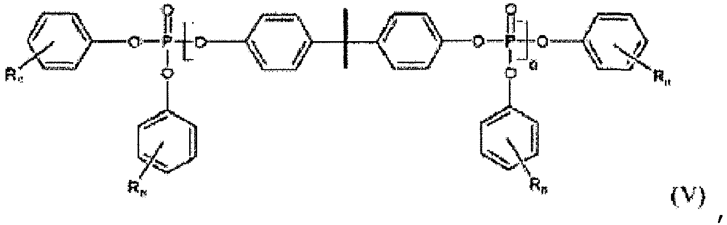 où chaque R est indépendamment l'hydrogène, un alkyle en C₁ à C₄, ou un hydroxy, n = 1 à 3 et o = 1 à 10.

14. Composition selon l'une des revendications 3 à 12, **caractérisée en ce que** l'au moins un composé de phosphore exempt de métal est un phosphonate-ester oligomère de la formule (VI) : 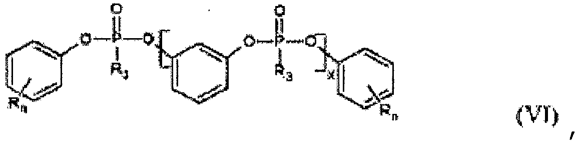 où R³ = méthyle ou phényle et x = 1 à 20, et R, n ont les significations indiquées dans la revendication 13.

15. Composition selon l'une des revendications 3 à 12, **caractérisée en ce que** l'au moins un composé de phosphore exempt de métal est un phosphonate-ester cyclique de la formule (VII) : 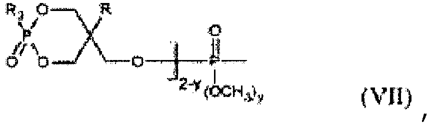 où y = 0 ou 2.

16. Composition selon l'une des revendications 3 à 12, **caractérisée en ce que** l'au moins un composé de phosphore exempt de métal est l'ester de l'acide thiopyrophosphorique de la formule (VIII):

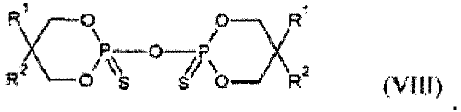

17. Composition selon l'une des revendications 3 à 12, **caractérisée en ce que** l'au moins un composé de phosphore exempt de métal est présent sous la forme d'un ester de phosphonite sous la forme de dérivé d'ester benzènemonophényle ou d'un dérivé de 9,10-dihydro-9-oxa-10-phosphaphenanthrene-10-oxyde (DOPO) ou d'un dérivé 6*H-*dibenzène(c,e) (1,2)-oxaphosphorin-6-one) de la formule (IX) ou (X) :

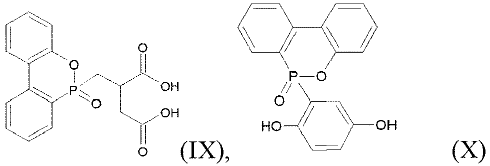

18. Composition selon l'une des revendications 3 à 17, **caractérisée en ce qu'**elle contient en plus des polyols et/ou des aminouraciles et/ou des composés POSS et/ou du tris-hydroxyéthylisocyanurate et/ou du cyanurate de mélanine et/ou du graphite expansé.

19. Composition selon la revendication 18, **caractérisée en ce que** la ol est le polyol pentaérythrite, dipentaérythrite, ou tripentaérythrite, l'aminouracil 1,3-diméthyl-6-aminouracile et le composé POSS est à base de méthylsiloxane.

20. Composition selon l'une des revendications 2 à 19, **caractérisée en ce que** la concentration de l'au moins un phosphate de métal à triazine intercalaire (a) et le composant (b) dans un polymère ou un mélange de polymères est de préférence de 0,1 à 60% en poids sur la base du polymère à traiter.

21. Utilisation d'une composition selon l'une des revendications 2 à 20 comme retardateur de flamme dans un polymère, du papier, un textile, ou des composites bois-matière plastique (WPC).

22. Utilisation selon la revendication 21, **caractérisée en ce que** le polymère est une matière thermoplastique qui est de préférence choisie dans le groupe comprenant les polyamides, les polycarbonates, les polyoléfines, le polystyrène, le polyester, le chlorure polyvinylique, l'alcool polyvinylique, l'ABS et le polyuréthanne, ou une résine thermodurcissable qui est de préférence choisie dans le groupe comprenant une résine époxy, une résine phénolique, et résine de mélamine.

23. Procédé de production d'un composé selon la revendication 1, comprenant l'étape de
réaction d'un composé (A), où (A) est une triazine de des formules (II -4), (II-5), ou (II-6) 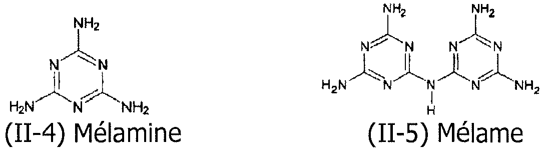 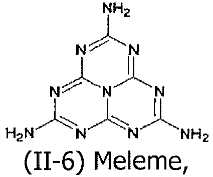 avec un phosphate de métal trinitruré de la formule Hₐ⁽⁺⁾[M_{b}^{m+}(H₂PO₄)ₓ₁⁽⁻⁾ (HPO₄)ₓ₂²⁽⁻⁾(PO₄)ₓ₃³⁽⁻⁾(PO₃)_{y}⁽⁻⁾]^{(a-)} * p H₂O, où chaque M = Al.

24. Procédé selon la revendication 23, **caractérisé en ce que** la réaction a lieu dans de l'eau et de préférence entre 20 et 90 °C, de manière particulièrement préférée entre 20 et 60 °C, et de façon tout particulièrement de préférence entre 20 et 40 °C.
